# EUROPEAN PATENT APPLICATION

(11) **EP 3 263 104 A1**
(43) Date of publication of application: **03.01.2018**
(21) Application number: 16177514.3
(22) Date of filing: 01.07.2016
(51) Int. Cl.: A61K 31/4545, A61P 9/04, A61P 11/00, A61P 27/02, A61P 25/00, A61P 25/02, A61P 43/00

(54) **USE OF BGP15 TO STIMULATE MITOCHONDRIAL FUSION**

(71) Applicant: N-Gene Research Laboratories Inc., New York, NY 10022 (US)
(72) Inventor: SUMEGI, Balazs, 7634 Pecs (HU); MANDL, Jozsef, 1132 Budapest (HU)
(74) Representative: Bianchetti Bracco Minoja S.r.l.

(57) **Abstract**

The present invention relates to the use of O-(3-piperidino-2-hydroxy-1- propyl)-nicotinic amidoxime (BGP15), its tautomers, enantiomers and pharmaceutically acceptable salts thereof the treatment of diseases characterised at least in part by an excessive mitochondrial fragmentation activity or by a reduced mitochondrial fusion activity such as pulmonary fibrosis, age related macular degeneration, retinitis pigmentosa, heart failure with preserved ejection fraction, an inherited mtDNA depletion disorder (such as Alper's disease) or an inherited mtDNA deletion/mutation disorder (such as progressive external ophthalmoplegia, an ataxia-neuropathy spectrum disorder or mitochondrial neurogastrointestinal encephalomyopathy).

## Description

The present invention relates to the use of O-(3-piperidino-2-hydroxy-1- propyl)-nicotinic amidoxime (BGP15), its tautomers, enantiomers and pharmaceutically acceptable salts thereof the treatment of diseases characterised at least in part by an excessive mitochondrial fragmentation activity or by a reduced mitochondrial fusion activity such as pulmonary fibrosis, age related macular degeneration, retinitis pigmentosa, heart failure with preserved ejection fraction, an inherited mtDNA depletion disorder (such as Alper's disease) or an inherited mtDNA deletion/mutation disorder (such as progressive external ophthalmoplegia, an ataxia-neuropathy spectrum disorder or mitochondrial neurogastrointestinal encephalomyopathy).

### Background of the invention

Mitochondria are dynamic organelles undergoing frequent fission and fusion cycles, and oxidative stress facilitates mitochondrial fragmentation and the destabilization of mitochondrial membrane system. Mitochondrial membrane dynamic are directed by dynamin type GTPases (Drp1, Mfn1/2 and OPA1), and from these dynamin type GTPases Drp1 is the main driving force of the fission, while Mfn1/2 responsible for mitochondrial outer membrane fusion and Opal is for the fusion of mitochondrial inner membranes (Zorzano *et al.,* 2015). Fragmented-mitochondria induce oxidative stress cell death and insulin resistance and contribute to the development and the progression of oxidative stress-related diseases (Nasrallah *et al.,* 2014 and Andreux *et al.,* 2013).

Since mitochondrial fragmentation plays an important role in the development and progression of several diseases, as set out in further detail below, compounds that reverse mitochondrial fragmentation can prove to be beneficial.

### PRIOR ART

The compound of formula I,

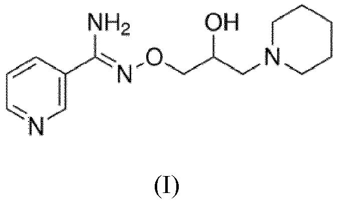

O-(3-piperidino-2-hydroxy-1- propyl)-nicotinic amidoxime, also known as BGP15, is known from WO2005123049 for increasing the number of mitochondria in various tissues. Nagy *et al.* (2010) describe how BGP15 prevents AIF mitochondria to nucleus translocation and mitochondrial depolarisation. Wu at al. (2015) describe how BGP15 increases mtDNA content in oocytes. Halmosi *et al.* (2001) and Szabados *et al.* (2000) show how BGP15 protects cardiac cells from reactive oxygen species (ROS) induced inactivation of mitochondria by virtue of its PARP-inhibitory action. Farkas *et al.* (2002), discusses the effect of BGP15 on skin mitochondria in relation to its anti-PARP activity.

### BRIEF DESCRIPTION OF THE DRAWINGS

Fig. 1A - Damage induced by Bleomycin on mice lungs treated or untreated with BGP15.
Fig. 1B - Hematoxylin eosin staining of on mice lung cells before and after treatment with BGP15, with or without bleomycin injury.
Fig. 1C - Alveolar thickness of the tissues of Fig. 1B.
Fig. 2A - Mac3 labelling of mice lung before and after treatment with BGP15, with or without bleomycin injury.
Fig. 2B - MAC3 counts of the tissues of Fig. 2A.
Fig. 3A - Mallory's trichrome staining of mice lung before and after treatment with BGP15, with or without bleomycin injury.
Fig. 3B - Ashcroft scores of the tissues of Fig. 3A.
Fig. 4A - α-SMA immunohistochemical staining of mice lung before and after treatment with BGP15 , with or without bleomycin injury.
Fig.4B - α-SMA density measured on the tissues of Fig. 4A.
Fig. 5A - 4-hydroxynonenal (HNE) immunohistochemical staining of mice lung before and after treatment with BGP15 , with or without bleomycin injury.
Fig. 5B - HNE density measured on the tissues of Fig. 5A.
Fig. 6A - Anti nitrotyrosine (NT) immunohistochemical staining of mice lung before and after treatment with BGP15 , with or without bleomycin injury.
Fig. 6B - NT density measured on the tissues of Fig. 6A.
Fig. 7A - Imaging of A549 mitochondria before and after treatment with H2O2, with or without treatment with BGP 15.
Fig. 7B - Counts of mitochondrial fragmentation on the samples of Fig. 7A.
Fig. 7C - Percentage of fused mitochondria in A549 cells before and after treatment with BGP15.
Fig. 8A - DHR123 to rhodamine123 imaging of RAW264 cells.
Fig. 8B - Counts of reactive oxygen species (ROS) production measured form Fig. 8A.
Fig. 9 - PARP-1 self-poly-ADP-ribosylations in mice lungs treated or untreated with BGP15.
Fig. 10 - Effect on RPE cell viability of various doses of BGP15 after Blue LED light induced RPE cell death.

### DETAILED DESCRIPTION OF THE INVENTION

We have surprisingly found that BGP15 promotes mitochondrial fusion and inhibits mitochondrial fragmentation and that this mechanism is independent form the known anti PARP activity of BGP15.

Accordingly, in a first aspect of this invention, there is provided the compound of formula I, its tautomers, stereoisomers and pharmaceutically acceptable salts thereof for the treatment of a disease characterised at least in part by a reduced mitochondrial fusion activity or by an excessive mitochondrial fragmentation activity.

In one embodiment the disease is characterised by an excessive mitochondrial fragmentation activity.

In another embodiment the disease is characterised by a reduced mitochondrial fusion activity.

### PULMONARY FIBROSIS

In one embodiment, the disease is pulmonary fibrosis.

We have surprisingly determined that BGP15 can be useful in the treatment of pulmonary fibrosis , that this effect is due to the surprisingly novel finding that BGP15 is able to inhibit mitochondrial fragmentation and that this property is independent of the known PARP inhibitory activity of BGP15, as set out in examples 1-7 below.

### Example 1 - Effect of BGP15 on pulmonary damage, macrophages infiltration and fibrosis.

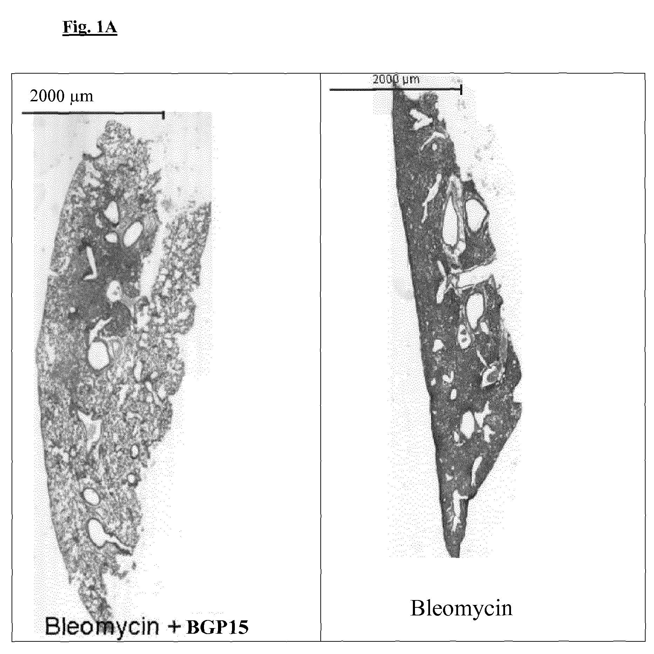

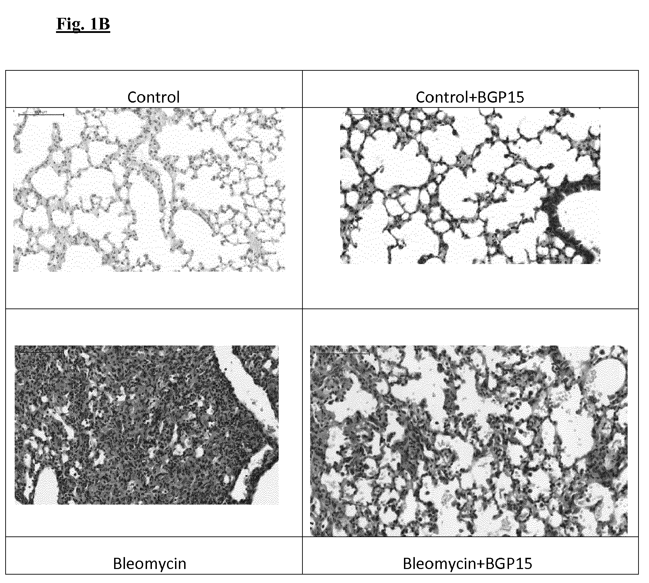

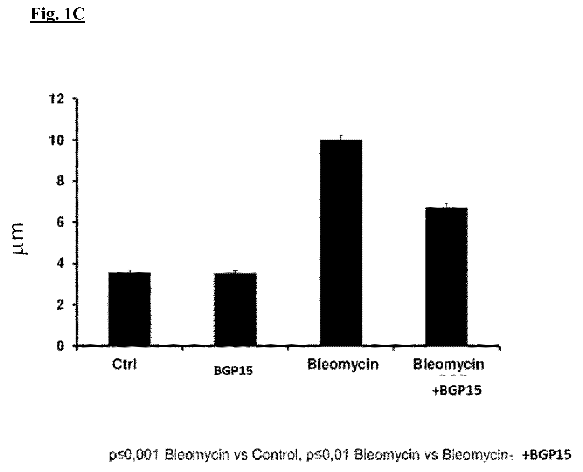

It is well-established that Bleomycin induces lung fibrosis in murine model, and Bleomycin induces airway epithelial cells damage, inflammation, fibroblast proliferation and differentiation, and extracellular collagen deposition in lung tissues (Kundu *et al.,* 2013; Fleishman *et al.,* 1971; Adamson *et al.,* 1974; Rossi *et al.,* 2004). Fig 1A shows that Bleomycin treatment inducing large scale lung damages in mice, while Bleomycin treatment together with BGP15 treatment significantly attenuates lung damages. The hematoxylin eosin staining shows thickness of alveolar sac was not significantly affected by BGP15 treatment (Fig. 1B, C). Bleomycin treatment caused inflammatory lesions, extensive vascular remodeling and interstitial fibrosis (Fig. 1B, C) and significantly increased the mean thickness of alveolar sac (*** p<0,05) (Fig. 1B, C). BGP15 treated pulmonary fibrosis (Bleomycin-treated) animal lungs show improved histological appearance, decreased infiltration with inflammatory cells, decrease in the thickness of alveolar sac (+++ p≤0.05) (Fig. 1B, C).

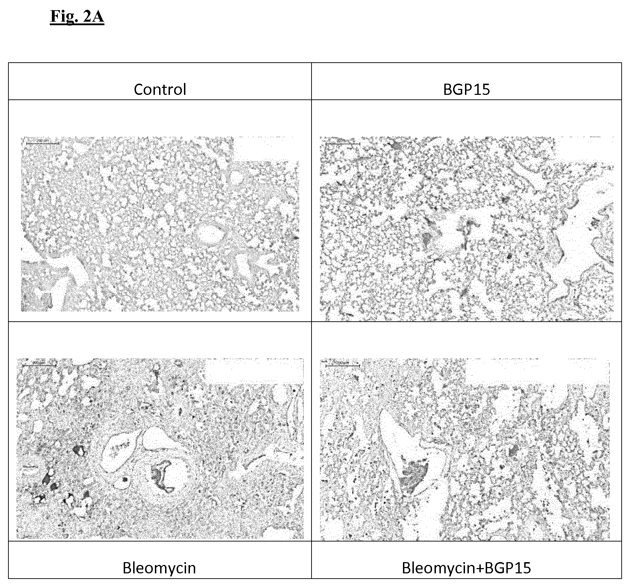

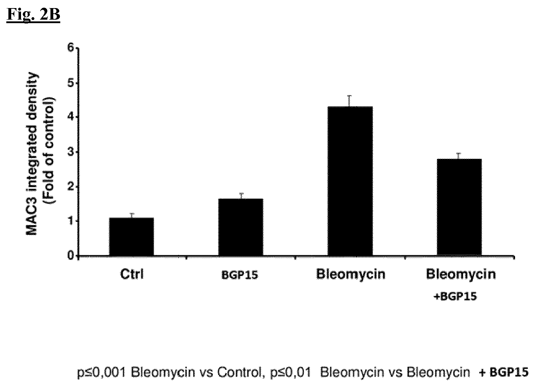

Bleomycin induced macrophages infiltration can be showed directly by labeling macrophages with Mac3 antibody. Fig. 2A, B show that large increases in MAC3 labeling in Bleomycin-treated mice lungs, and that BGP15 treatment (together with Bleomycin) significantly reduces macrophages infiltration into Lung parenchyma. These data show that BGP15 has significant protective effect against Bleomycin-induced lung parenchyma damage and prevents macrophages infiltration.

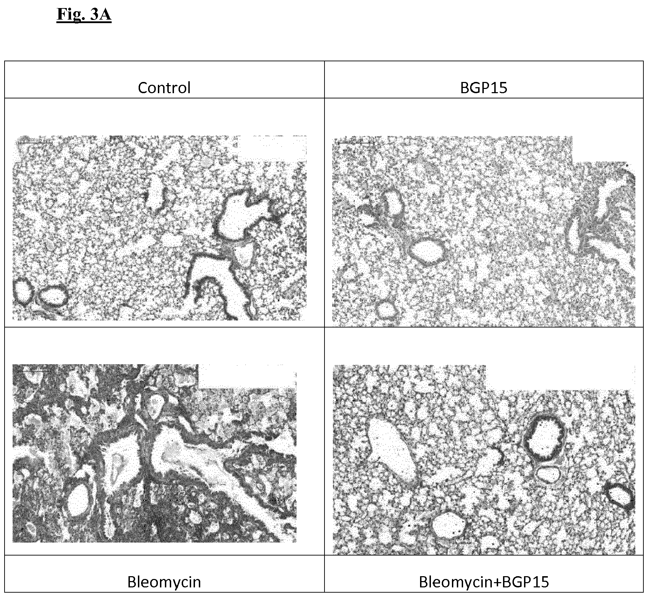

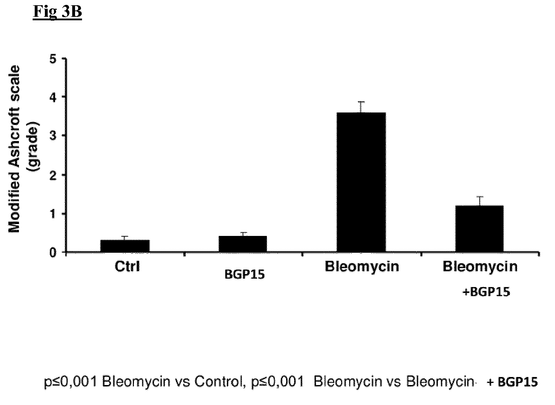

Determining the protective effects of BGP15 in bleomycin-induced lung fibrosis we used Mallory's trichrome staining to assess collagen deposition in lung tissues in Bleomycin-treated mice. Control lung sections and alone BGP15-treated lung sections showed a small amount of collagen fibers (stained blue) in the alveolar septum (Fig. 3A). Lung sections of Bleomycin-treated mice showed more collagen depositions and fibrotic lesions, but giving BGP15 together with Bleomycin significantly reduces the collagen deposits and lesions (Fig. 3A). Ashcroft score were used to assess the severity of lung fibrosis, and to compare Control, BGP15 treatment, Bleomycin-treatment and Bleomycin + BGP15-treatments (Fig. 3B) , and these data show the development of fibrosis in Bleomycin treated mice and that BGP15 significantly reduces lung fibrosis (Fig. 3B).

### Example 2 - Effect of BGP15 on the expression of α-SMA in pulmonary fibrosis model system.

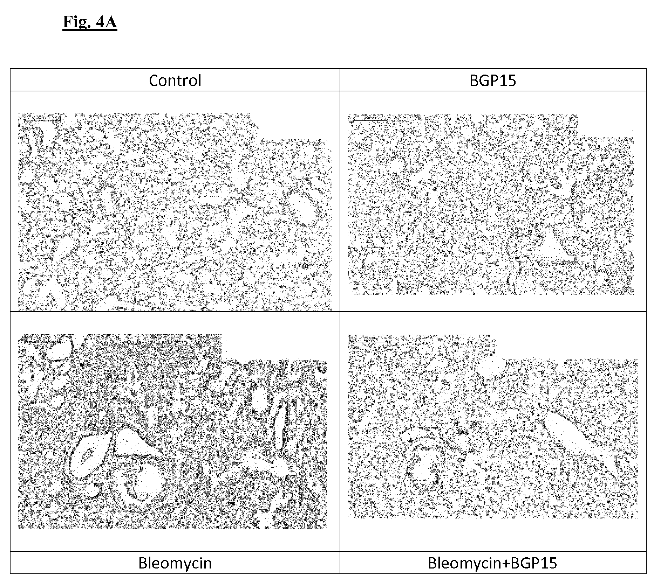

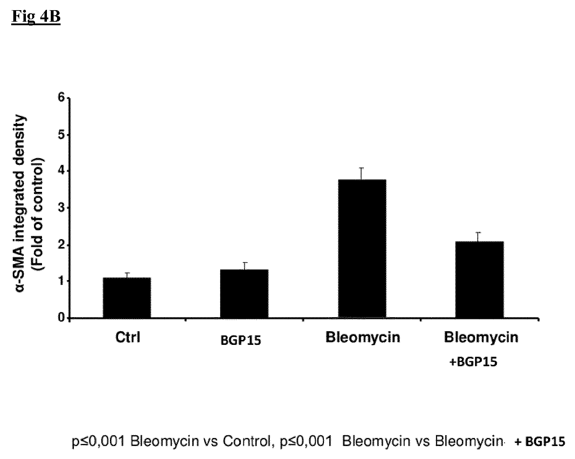

Inflammatory processes activate the formation of myofibroblast from fibroblast which is characterized by α-SMA production (Maarsingh *et al.,* 2008). We provided evidence for the extent of myofibroblast formation and fibrosis in lung sections by determining α-SMA with immunohistochemical staining (Fig. 4A, B). Significant increases in the levels of α-SMA and increased number of α -SMA-positive cells are detected in lung tissues from Bleomycin-treated mice compared to control lungs and lungs deriving from BGP15 treated cells (Fig. 4A, B). Comparing Bleomycin-treated mice with Bleomycin plus BGP15 treated mice show that BGP15 significantly reduced α-SMA levels and reduced numbers of α-SMA-positive cells (Fig. 4A, B). These data (Fig. 4A, B), suggests that BGP15 inhibits myofibroblast activation in Bleomycin-induced lung fibrosis model.

### Example 3 - Effect of BGP15 on lipid peroxidation and on nitrotyrosine formation.

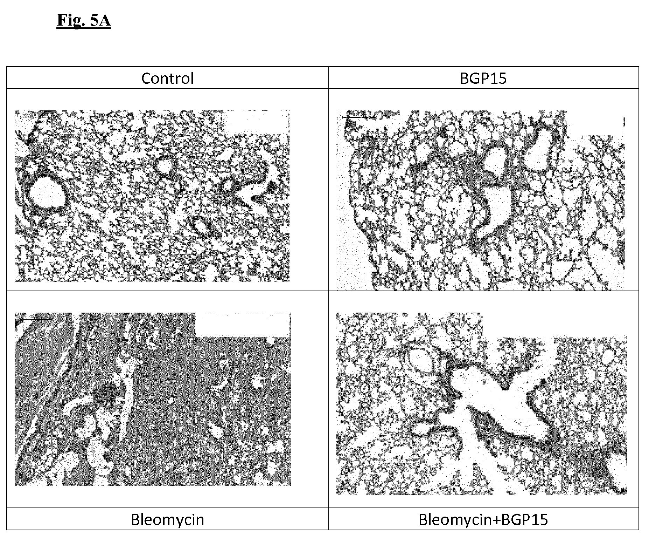

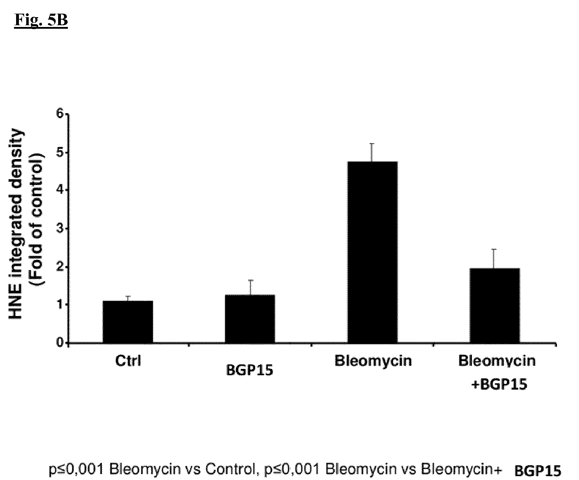

It is well-known that oxidative/nitrosative stress plays an important role in the pathogenesis of pulmonary fibrosis. Oxidative/nitrosative stress can induce lipid peroxidation which can be characterized by the formation of 4-hydroxynonenal, and 4-hydroxynonenal can reacts to proteins and its product can be determined by immunohistochemistry using anti-4-hydroxynonenal antibody. Fig. 5A, B, shows lipid peroxidation from 4-hydroxynonenal production using immunohistochemistry, and these data shows elevated lipid peroxidation in Bleomycin-induced lung fibrosis compared to untreated, or BGP15 treated mice. However, the addition of BGP15 to Bleomycin-treated mice significantly reduced 4-hydroxynonenal formation and so lipid peroxidation compared to bleomycin treated mice (Fig. 5A, B). These data show (Fig. 5A, B), that BGP15 significantly reduces lipid peroxidation in bleomycin induced lung fibrosis model.

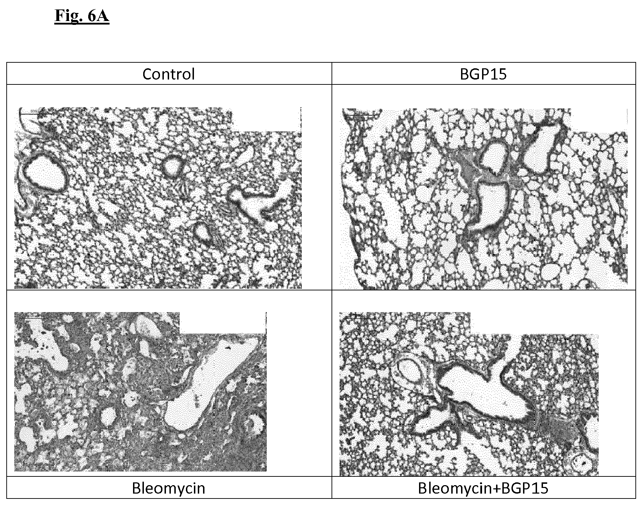

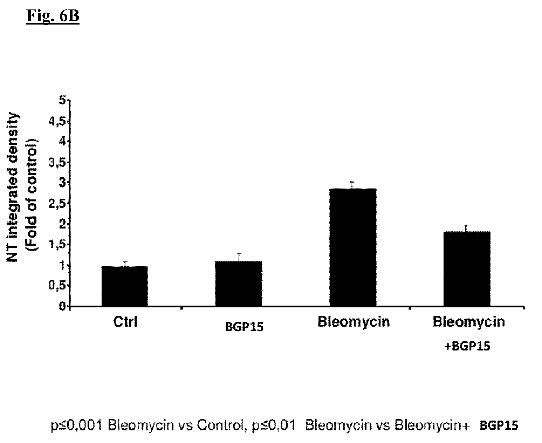

NO and peroxynitrite play an important role in lung pathogenesis and NO reacts with superoxide to produce peroxynitrite which nitrosylates proteins (Maarsingh *et al.,* 2008). Therefore, nitrosative stress enhances the presence of nitrotyrosine residues in proteins which can be measures by protein nitrotyrosine quantity by anti-nitrotyrosine antibody by immunohistochemistry (Fig. 6A, B). Bleomycin induces the formation of protein nitrotyrosine residues, comparing to control lungs (Fig. 6A, B), but giving BGP15 together Bleomycin reduces nitrotyrosine formation, showing again that BGP15 reduces oxidative/nitrosative stress in Bleomycin-induced lung fibrosis model.

### Example 4 - BGP15 prevents the oxidative stress induced mitochondrial fragmentation in lung epithelial cells.

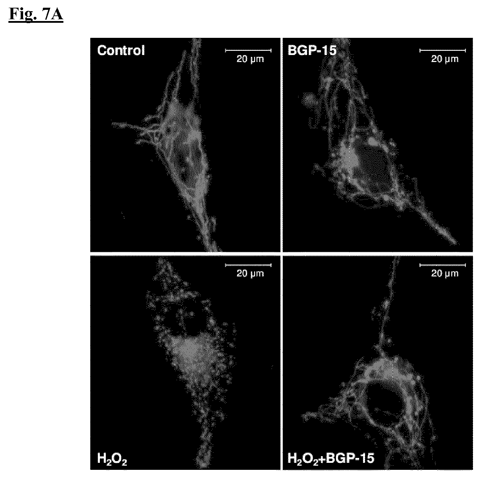

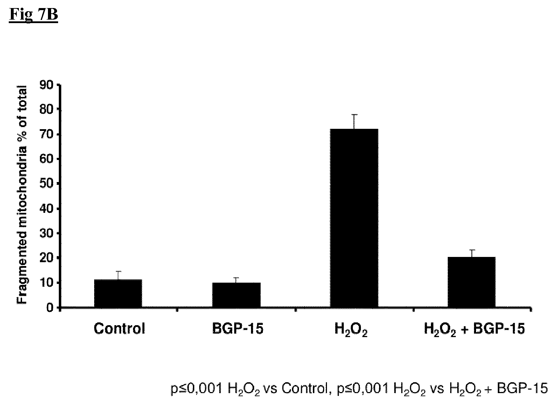

Epithelial cells participate in the pathogenesis in pulmonary fibrosis, and they are damaged by oxidative stress which can lead to mitochondrial fragmentation. A-549 cells (lung epithelial tumor cell) have many long mitochondrial filaments under normal conditions (Figs. 7A), and the induction of mitochondrial fragmentation with 50 µM H₂O₂ for 5 hours. Mitochondrial fragmentation was induced in A-549 cells with 50 µM H₂O₂ for 5 hours, and we found that mitochondrial filaments disappeared, and predominantly fragmented mitochondria with lengths shorter than 2 µm were seen (Figs. 7A, B). Addition of 50 µM H₂O₂ to A-549 cells in the presence of 50 µM BGP15 could induce only very limited amount of fragmentation showing that BGP15 protects mitochondria against H₂O₂-induced fragmentation (Figs. 7A, B). These data show that BGP15 by preventing Reactive Oxygen Species (ROS) induced mitochondrial fragmentation can prevent lung fibrosis at mitochondrial level.

### Example 5 - BGP15 promotes fusion in A549 cell lines

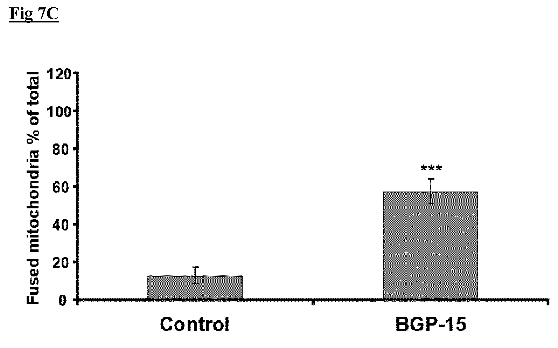

A549 cells were transfected with mitochondrial directed red or green fluorescent proteins (mARFP or mAGFP), and were co-cultured and fused by the PEG-fusion method. After the removal of PEG, cells were cultured for 2 hours, fixed and then mitochondrial fusions were detected by fluorescent microscopy (Fig. 7C). Fluorescent microscopic pictures indicate some degree of mitochondrial fusion (from the migration of red or green fluorescent proteins) in control cells; however, BGP15 treatment significantly increased fusion, as seen by red or green migration rates from fluorescent microscopic pictures (Fig. 7C). While the fusion of red- and green-labeled proteins only extend to the closest 10-20 % of the next cells in control experiments, the mitochondria-directed fluorescent molecules get to the opposite side of BGP15-treated cells, suggesting that BGP15 directly promotes the rate of mitochondrial fusion.

### Example 6-BGP15 reduces lipopolysaccharide (LPS) induced mitochondrial ROS production in RAW264 macrophages.

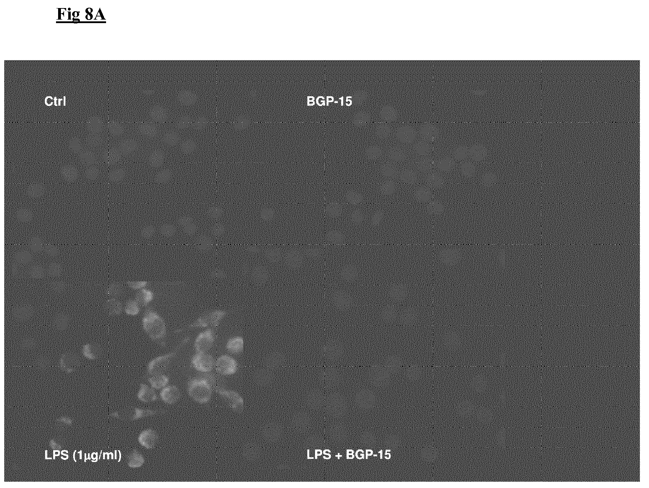

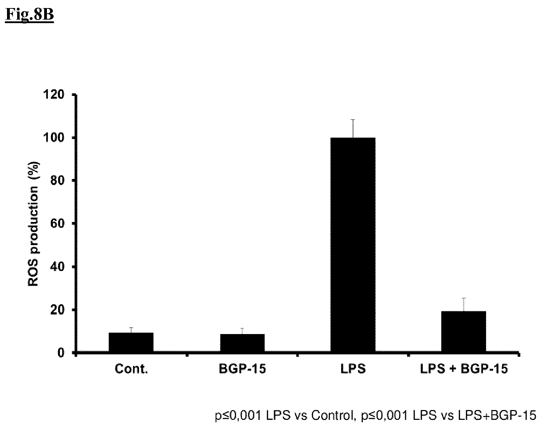

Pulmonary macrophages play a significant role in the initiation and disease progression in pulmonary fibrosis, and involved in inflammatory response and oxidative stress. We investigated the effect of BGP15 on LPS-induced ROS production using RAW 264 macrophages which has an active LPS-TLR4 signaling pathway. It is well-known that LPS induces mitochondrial ROS production by raising cytoplasmic calcium level (Zanoni *et al.,* 2009), therefore this is a different mechanism compared to the only H₂O₂ induced mitochondrial ROS production. Fig. 8A and B. Show that LPS induces mitochondrial ROS production detected by the oxidation of DHR123 to rhodamine123 (green fluorescence) while in the control, or control + BGP15 treated cells, only very minimal amount ROS production was detected. BGP15-treatment diminishes LPS-induced ROS production in RAW 264 macrophages cells showing that BGP15 attenuates mitochondrial ROS production independently of the initiation mechanism. Quantitative data (Fig. 8B). support our observation in microscopic pictures (Fig. 8A).

### Example 7 - Effect of BGP15 on poly-ADP-ribosylation in lung tissues of rats in pulmonary fibrosis.

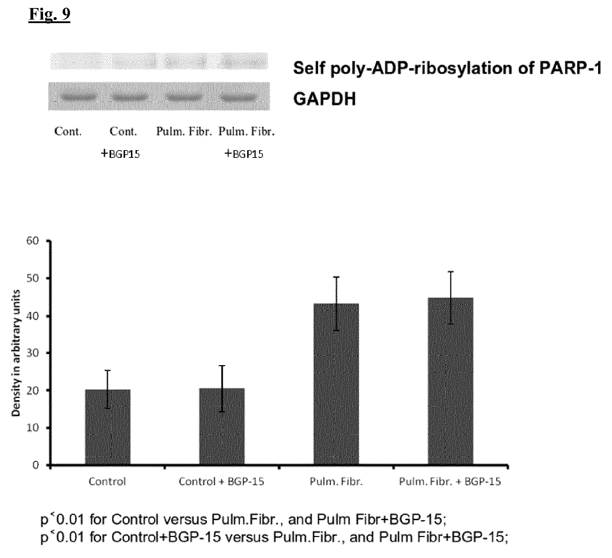

It was showed before (Szabados *et al.* (2000)) that BGP15 at high concentration can inhibit PARP-1 *in nitro.* Under our experimental conditions in bleomycin induced pulmonary fibrosis model, PARP-1 self-poly-ADP-ribosylations were increased in pulmonary fibrosis comparing to control lung, but BGP15 treatment did not have any effect on PARP-1 self-poly-ADP-ribosylations neither in pulmonary fibrosis lung tissues nor in control lung tissues (Fig.9). These data show that BGP15 protects lung tissues by other mechanism, not by regulating PARP activity in pulmonary fibrosis *in vivo* in lung tissues.

### AGE RELATED MACULAR DEGENERATION AND RETINITIS PIGMENTOSA

In one embodiment, the disease is age-related-macular degeneration.

In another embodiment the disease is retinitis pigmentosa.

Indeed, we have surprisingly determined that BGP15 rescues the apoptotic effect of blue LED light on Retinal Pigment Epithelium (RPE), as set out in example 8 below.

Retinal degeneration as seen in age-related macular degeneration (AMD) and retinitis pigmentosa (RP), is the most common form of neural degenerative disease in the world and the leading cause of impaired vision and registered blindness in adults. (Buch *et al.,* 2004; Al-Merjan *et al.,* 2005; Hata *et al.,* 2003; Frick *et al.,* 2012; Wert *et al.,* 2014). RP is a hereditary disease which is characterized by night blindness and constriction of peripheral visual fields, and eventually results in severe visual disturbance. (Wert *et al.,* 2014) RP is prevalent in inherited retinal dystrophy and along with age-related macular degeneration (AMD) (Hata *et al.,* 2003) which are a devastating threat to public vision health worldwide. (Chang *et al.,* 2014) AMD is more common in the elderly in developed countries and is prone to racial differences in prevalence (Chang *et al.,* 2014) Degenerative loss of photoreceptors occurs in inherited and age-related retinal degenerative diseases. (Chang *et al.,* 2014) In the early stages of the disease, the rod photoreceptors degenerate, which are followed by the dysfunction of the cone photoreceptor. Molecular genetic studies have revealed that at least 47-50 genes are involved in or cause RP there are currently no effective treatments for RP. (Ozaki *et al.,* 2012) Treatment options are limited for these conditions so a lot of effort has been put into delaying progressive vision deterioration which is a characteristic of these diseases (Chang *et al.,* 2014) Light is an environmental risk factor for RP and AMD because it can accelerate the progression of RP (Paskowitz *et al.,* 2006). In animal models with rhodopsin mutations of human autosomal dominant RP there is slower recovery of rod function and greater degree of photoreceptor death by apoptosis in the inferior retina after light exposure (Kijas *et al.,* 2002; Naash *et al.,* 1996; Wenzel *et al.,* 2005). Studies have shown that light-induced retinal damage in albino rats manifests histopathology and patterns of differential expression of disease-associated genes similar to those found in atrophic AMD (Marc *et al.,* 2008; Rutar *et al.,* 2010; Rutar *et al.,* 2011). It has been shown that intense white light exposure causes death of mouse-derived photoreceptors (661W cells) by both necrosis and death receptor Fas-mediated apoptosis (Chang et al, 2014 and 2015). Mitochondrial µ-calpain initiates apoptosis-inducing factor (AIF)-dependent apoptosis in retinal photoreceptor degeneration and Wang *et al.* (2015) describe how calpain is a critical component of mitochondrial fragmentation.

Moreover, Plafker *et al.* (2012) describe how mitochondrial fragmentation is involved in RPE damage.

### Example 8 - Effect of BGP15 on the blue LEDs light exposure on the death of RPE cells.

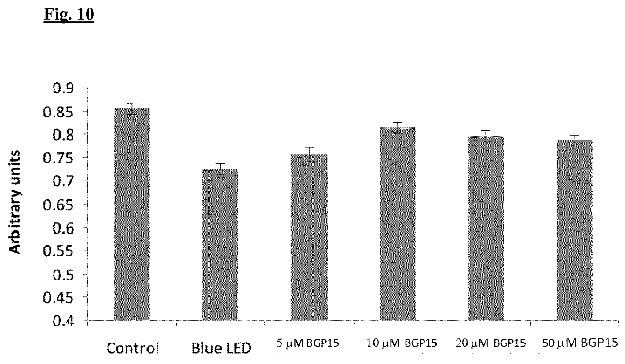

Blue LED light induced cell death were studied on Retinal Pigment Epithelium (RPE) cells, showing that 4 hour light exposure induced detectable amount cell death determined by the sulforhodamine method (Fig. 10). The effect of BGP15 in the 5-50 µM range was also analyzed and Fig. 10 shows that BGP15 has protective effect and the optimal concentration is 10 µM.

### HEART FAILURE WITH PRESERVED EJECTION FRACTION

In one embodiment, the disease is heart failure with preserved ejection fraction.

There is evidence that mitochondria and mitochondrial ROS production are very important in the progression of the heart failure patients with preserved ejection fraction (HFpEF) (Schwarzer et al, 2014). ROS production peaked at a few weeks of pressure overload, and mitochondrial respiratory capacity (state 3 respiration) was elevated after transverse aortic constriction, and decreased after a certain point and significantly decreased at 20 weeks, when contractile function was also impaired and ROS damage was found with increased hydroxynonenal. In addition, cardiac mitochondrial ROS production induced the development of pressure overload-induced heart failure (Schwarzer et al, 2014). Other findings demonstrated that HFpEF induces significant molecular, mitochondrial, histological, and functional alterations (Bowen *et al.,* 2015). In addition, it was shown in different studies that Coenzyme Q10, which carries hydrogen between the mitochondrial respiratory complexes has protective effect in HFpEF (Sharma *et al.,* 2016).

BGP15, therefore, because it activates mitochondrial fusion, can be used for the treatment of HFpEf.

### INHERITED mtDNA MUTATION/DELETION OR DEPLETION DISEASES.

It has been shown that active mitochondrial fusion is required for mitochondrial genome maintenance and mtDNA replication (Elachouri *et al.,* 2011 and Hori *et al.,* 2011) Therefore, activation of mitochondrial fusion can contribute to better mtDNA replication.. Up to now, there are only lower eukaryotic systems when certain mtDNAs can be specifically eliminated by the joint process of mitochondrial fusion, fission and mitophagy (Mishra *et al.,* 2014).

Therefore, activation of mitochondrial fusion in synergy with mitophagy can differentiate between small mitochondrial vesicles with good mtDNA and high membrane potential and small mitochondrial vesicle with mutated mtDNA and low membrane potential. It is well-known that high level mitochondrial mutations/deletion result in compromised respiratory activity and depolarized mitochondria (Szczepanowska *et al.,* 2012 and Arduini *et al.,* 2011) providing basis of this selection mechanism, and that mitochondrial depolarization facilitates mitochondrial fission and mitophagy (Redmann *et al.,* 2014) In addition, mitochondrial fusion requires high mitochondrial membrane potential (Papanicolaou at al., 2012), therefore small mitochondrial vesicles with mutated/deleted mtDNA have low membrane potential (Szczepanowska *et al.,* 2012) and cannot be fused, and are thus directed to decomposition by mitophagy.

Because BGP15 stimulates mitochondrial fusion, it can thus be used for the treatment of inherited mtDNA deletion/mutation/depletion disorders such as Alper's disease (Darin *et al.,* 2001 and Young *et al.,* 2016), progressive external ophthalmoplegia (Meersseman *et al.,* 2014; Lestienne *et al.,* 1994; Paramasivam *et al.,* 2016), mitochondrial neurogastrointestinal encephalomyopathy (Young *et al.,* 2016 and Mihaylova *et al.,* 2013) or an ataxia-neuropathy spectrum disorder (Young *et al.,* 2016 and Bostan *et al.,* 2012).

Accordingly,
In one embodiment, the disease is an inherited mtDNA depletion disorder.

In a particular embodiment, the inherited mtDNA depletion disorder is Alper's Disease.

In another embodiment, the disease is an inherited mtDNA deletion/mutation disorder.

In particular embodiment, the inherited mtDNA deletion/mutation disorder is progressive external ophthalmoplegia.

In another particular embodiment, the inherited mtDNA deletion/mutation disorder is an ataxia-neuropathy spectrum disorder.

In yet another particular embodiment, the inherited mtDNA deletion/mutation disorder is mitochondrial neurogastrointestinal encephalomyopathy.

### FORMULATION AND ADMINISTRATION

BGP15 is formulated preferably in admixture with a pharmaceutically acceptable carrier, excipient or the like. In general, it is preferable to administer the pharmaceutical composition in orally-administrable form, but certain formulations may be administered via a parenteral, intravenous, intramuscular, transdermal, buccal, subcutaneous, suppository, nasal or other route. One of ordinary skill in the art may modify the formulations within the teachings of the specification to provide numerous formulations for a particular route of administration without rendering the compositions of the present invention unstable or compromising their therapeutic activity. In particular, the modification of the present compounds to render them more soluble in water or other vehicle, for example, may be easily accomplished by minor modifications (salt formulation, esterification, etc.) which are well within the ordinary skill in the art. It is also well within the routineer's skill to modify the route of administration and dosage regimen of a particular compound in order to manage the pharmacokinetics of the present compounds for maximum beneficial effect in patients. In certain pharmaceutical dosage forms, the pro-drug form of the compounds, especially including ester and ether derivatives, as well as various salt forms of the present compounds, are preferred. One of ordinary skill in the art will recognize how to readily modify the present compounds to pro- drug forms to facilitate delivery of active compounds to a targeted site within the host organism or patient. The routineer also will take advantage of favourable pharmacokinetic parameters of the pro-drug forms, where applicable, in delivering the present compounds to a targeted site within the host organism or patient to maximize the intended effect of the compound. Actual methods of preparing such dosage forms are known, or will be apparent, to those skilled in this art; for example, see Remington's Pharmaceutical Sciences, Mack Publishing Company, Easton, Pennsylvania, 15th Edition, 1975. The composition or formulation to be administered will, in any event, contain a quantity of the active compound in an amount effective to alleviate the symptoms of the subject being treated. While human dosage levels have yet to be optimized for the compounds of the invention, generally, a daily dose is from about 0.05 mg/kg to about 100 mg/kg of body weight. The amount of active compound administered will, of course, be dependent on the subject and disease state being treated, the severity of the affliction, the manner and schedule of administration and the judgment of the prescribing physician. For purposes of the present invention, a prophylactically or preventive effective amount of the compositions according to the present invention (i.e., an amount which substantially reduces the risk that a patient will either succumb to a disease state or condition or that the disease state or condition will worsen) falls within the same concentration range as set forth above for therapeutically effective amounts and is usually the same as a therapeutically effective amount. In some embodiments of the present invention BGP15 is administered in combination with one or more other pharmaceutically active agents. The phrase "in combination", as used herein, refers to agents that are simultaneously administered to a subject. It will be appreciated that two or more agents are considered to be administered "in combination" whenever a subject is simultaneously exposed to both (or more) of the agents. Each of the two or more agents may be administered according to a different schedule; it is not required that individual doses of different agents be administered at the same time, or in the same composition. Rather, so long as both (or more) agents remain in the subject's body, they are considered to be administered "in combination".

### MATERIALS AND METHODS

### PULMONARY FIBROSIS

All chemicals for cell culture studies were from PAA Laboratories (Cölbe, Germany) and Gibco/Invitrogen (Carlsbad, CA). Hydrogen peroxide and all remaining chemicals were purchased from Sigma Aldrich Co. (Budapest, Hungary). Fluorescent dye MitoTracker Red, Dihydrorhodamine 1,2,3 and Hoechst 33342 were obtained from Molecular Probes (Carlsbad, CA, USA). BGP15 (O-(3-pyperidino-2-hydroxy-1-propyl) pyridine-3-carboxylic acid amidoxime monohydrochloride) was a gift from Ngene (New York). Bleomycin (Bleomedac) was from Medac GmbH (Hamburg, Germany). All reagents were of the highest purity commercially available.

### Cell cultures

RAW 264 (mouse leukaemic monocyte-macrophage) and A549 (human lung carcinoma) cell lines were obtained from the European Collection of Cell Cultures (Salisbury, UK). The cell lines were maintained in a humidified 5% CO₂ atmosphere at 37°C. RAW 264 cells were cultured in Eagle's minimum essential *medium (MEM)* and A549 cells in Dulbecco's modified Eagle's medium (DMEM, PAA Laboratories, Cölbe, Germany). All media contained 10% bovine serum and antibiotic solution (1% penicillin and streptomycin mixture) (Gibco/Invitrogen, Carlsbad, CA). Cells were passaged at 3-day intervals.

### Animals

Male, C57BL/6 black mice 8-10 weeks of age and 25-30 g of body weight, obtained from Charles River Laboratories (Sulzfeld, Germany) were used. Animals were maintained in a 12:12-h light-dark cycle with food and water ad libitum. The mice were acclimated to the laboratory conditions for at least 1 week prior to the commencement of the experiments. All experiments were carried out in accordance with international guidelines of the care and use of the laboratory animals. Animals got freshly made solutions every day.

### Cell viability assay

The cells were seeded into 96-well plates at a starting density of 10⁴ cells per well and cultured overnight before Bleomycin and BGP15 added to the medium for 48 h. The medium was changed to a fresh one containing 0.5% of 3-[4,5-dimethylthiazol-2-yl]-2,5-diphenyl-tetrazolium bromide (MTT⁺) for an additional 3 hours, then the MTT⁺ reaction was terminated by adding HCl to 10 mM final concentration. The amount of blue formazan dye formed from MTT⁺ was proportional to the number of live cells, and was determined with GloMax Multi Detection System (Promega, USA) at 560 nm. All experiments were run in at least 4 replicates and repeated 3 times.

### Determination of intracellular reactive oxygen species

Intracellular ROS were determined using the oxidation-sensitive Dihydrorhodamine123 (DHR) fluorescent dyes. Cells were seeded at a starting density of 1x10⁵ cells/well for fluorescent microscopy. After subjecting the cells to the treatment indicated in the figure legends, medium was replaced to a fresh one containing 2 µg/ml DHR. Incubation was continued for an additional 15 minutes to allow oxidation of DHR by the endogenous ROS. Fluorescence of oxidized DHR was excited at 485 nm and the evoked emission was measured at 525 nm by Fluorescent microscope.

### Induction of pulmonary fibrosis

To induce pulmonary fibrosis, animals were tracheostomized under anesthesia by intra-peritoneal (i.p.) injection of 75 mg/kg of ketamine. Then bleomycin hydrochloride was administered as a single dose of 3 mg/kg in 50 µl of sterile saline intratracheally (i.t.) Control mice received an equal volume of sterile saline intratracheally. Ten animals were used in each control and experimental groups. After intratracheal instillation of bleomycin, BGP15 (50mg/kg in 200 µl of sterile saline) or equal volume of sterile saline was administered intraperitoneally once a day for 14 days.

### Lung histology

Two weeks after instillation of bleomycin, animals were sacrificed by a high dose of ketamine. Lungs were removed and then weighed. Samples of left lung were taken for biochemical analysis of collagen content. Samples of left lung were fixed, processed and 4-µm-thick paraffin embedded sections were used for immuno-histochemical, haematoxylineosin (H&E) and Mallory's trichrome staining and digital photos were taken. Average wall thickness of alveolar sac was determined at randomly chosen 25 different sites in each section by an expert who was blind to the experiment. Macrophages were counted in 5 non-overlapping high power fields (200x) in each sections by an expert who was blind to the experiments.

Semiquantitative morphological study of pathological changes in the lung sections was carried out to assess the severity of pulmonary fibrosis. These changes were graded according to the method described by Ashcroft and coworkers in a blinded fashion on a scale of 0 to 8 by examination of 10 randomly chosen regions per sample at a magnification of × 100. These grades are as follows: grade 0=normal tissue; grade 1=minimal fibrous thickening of alveolar or bronchial walls; grade 3=moderate thickening of walls without obvious damage to the lung architecture; grade 5=increased fibrosis with definite damages to the lung structure and formation of fibrous bands or small fibrous masses; grade 7=severe distortion of structure and large fibrous areas; grade 8=total fibrous obliteration of the field (Ashcroft *et al*.,1988).

### Immunohistochemical staining

Slides were deparaffinized in xylene, rehydrated in graded ethanol series, and washed in distilled water. Heat induced epitope retrieval was performed by boiling the tissue sections in citrate buffer (HISTOLS Citrate Buffer, Histopathology Ltd.) in a microwave oven at 750 W followed by cooling at room temperature for 20 minutes. Slides were washed in tris buffered saline (TBS) solution (pH = 7,6) followed by blocking of endogenous peroxidase (Peroxidase blocking, Histopathology Ltd.) for 10 minutes at room temperature. Slides were washed in TBS. Nonspecific sites were blocked (Background Blocking Protein Solution, Histopathology Ltd.) for 10 minutes at room temperature. Without washing, the following primary antibodies were applied: Anti-Nitrotyrosine (Millipore, in 1:100 dilution) rabbit polyclonal antibody, Anti-4 Hydroxynonenal (1:200 dilution) rabbit polyclonal antibody, Anti-Macrophage Marker (MAC3, in 1:50 dilution) rabbit polyclonal antibody, Anti-alpha smooth muscle Actin (α-SMA, Abcam, in 1:50 dilution) rabbit polyclonal antibody. Incubation with the primary antibodies was performed for 1 hour at room temperature followed by washing in TBS. Secondary antibody (HISTOLS -R Detection System, anti-rabbit, Histopathology Ltd.) was applied for 30 minutes at room temperature followed by repeated washing in TBS. Sections were incubated with 3-amino-9-ethylcarbazol (HISTOLS -Resistant AEC Chromogen/Substrate System, Histopathology Ltd.) or 3,3 9 -Diaminobenzidine (HISTOLS DAB Chromogen/Substrate System, Histopathology Ltd.), washed in distilled water, counterstained with haematoxylin followed by incubation in tap water. Negative control was incubated with antibody diluent instead of the primary antibody and applying anti-rabbit secondary antibody. Sections were then dehydrated, cleared in xylene and mounted with permanent mounting medium.

### Data analysis

The resulting data are expressed as mean ± standard deviation. Data were analyzed using the Kolmogorov-Smirnov normality test followed by the one-way Anova test and Bonferroni post hoc multiple comparison test. Differences were considered significant for p <0.05. Analyses were performed using Excel 2013 and IBM SPSS Statistics 20.

### Effect of BGP15 on the PARP activation in pulmonary fibrosis.

Lung samples were homogenized in ice-cold isotonic Tris buffer (50 mM, pH 8.0) containing 0.5 mM sodium metavanadate, 1 mM EDTA, and a protease inhibitor cocktail (1:1000; Sigma-Aldrich) as described previously [50]. Proteins and poly-ADP-ribosylation (PARylation) were determined from the tissue homogenates. Lung tissue proteins were precipitated by trichloroacetate, washed three times with 220 µl acetone, dissolved in Laemmli sample buffer, separated on 12% SDS-polyacrylamide gels, and transferred to nitrocellulose membranes. After blocking with 3% nonfat milk in Tris-buffered saline for 2 h, membranes were probed overnight at 4 uC with antibodies recognizing Acting and poly-ADP-ribose monoclonal antibody which was a gift from Laszlo Virag (Debrecen, Hungary). The membranes were washed six times for 5 min in Tris-buffered saline (pH 7.5) containing 0.2% Tween before addition of goat anti-rabbit horseradish peroxidase-conjugated secondary antibody (1:3000 dilution; Bio-Rad). The protein bands were visualized with enhanced chemiluminescence labeling using an ECL immunoblotting detection system (Amersham Biosciences). Developed films were scanned and the pixel volumes of the bands were determined using the NIH ImageJ software, with the values in ratios of intensity. Each experiment was repeated a minimum of three times.

### PEG fusion assay

The PEG fusion assay was performed as described before (Giacomotto *et al.,* 2013 and Lovy *et al.,* 2012). Briefly, A549 cells were transiently transfected with mitochondria directed enhanced red (mERFP) or green (mEGFP) fluorescent protein. The next day, the PEG fusion assays were then performed. A549 cells expressing mERFP were co-plated with A549 cells expressing mEGFP on glass coverslips. Cycloheximide (20 µg/ml) was added 30 min before fusion and kept in all solutions used subsequently to inhibit protein synthesis. The 70-100% confluent cells in a 35-mm culture dish were then washed with minimal essential medium (DMEM) without serum and incubated for exactly 1 min with 750 µl of a prewarmed (37°C) solution of PEG 1500 (50% [wt/vol] in DMEM, Sigma Aldrich Co., Budapest, Hungary). After the removal of the PEG, the cells were washed 3 times with DMEM containing 10% serum and incubated with 50 microM BPG-15 for 2 hours. The cultures were fixed in 4% formalin, and the cells were visualized by Nikon Eclipse Ti-U fluorescent microscope equipped with a Spot RT3 camera using a 60x objective and epifluorescent illumination.

### AGE RELATED MACULAR DEGENERATION AND RETINITIS PIGMENTOSA

Human RPE cells were acquired from (Lonza (www.lonza.com*) and* were grown in Dulbecco's high glucose Modified Eagle Medium (Gibco, BRL Life Technologies, Paisley, UK) containing 3% fetal bovine serum (Gibco), 1% L-glutamine and 1% penicillin / streptomycin(all from Invitrogen-Gibco BRL, Paisley, UK). The cells were maintained in a humidified incubator at 37_C, 5% CO2 and were passed every 3 days. Human RPE cells were at an initial density of 2 10⁴ cells/well and allowed to attach for one day and irradiated by Blue-LED. Cell viability we determined by sulforhodamine method (Cheng *et al.,* 2013).

### REFERENCES

Adamson IY et al., Am J Pathol 1974; 77: 185-197.
Al-Merjan JI et al., (2005) Ophthalmic Epidemiol. 12, 251-257.
Andreux PA et al., Nat Rev Drug Discov. 2013 Jun;12(6):465-83.
Arduini et al., J. Am J Physiol Gastrointest Liver Physiol. 2011 Jul;301(1):G119-27.
Ashcroft et al., J Clin Pathol 1988; 41: 467-470.
Bostan A et al., Auton Neurosci. 2012 Sep 25;170(1-2):70-2.
Bowen et al., Eur J Heart Fail. 2015 Mar;17(3):263-72.
Buch H et al., (2004) Ophthalmology 111, 53-61.
Chang et al., J Biol Chem. 2014 Mar 21;289(12):8337-52.
Cheng et al., Acta Pharmacol Sin. 2013 Jul;34(7):951-9.
Darin et al., Ann Neurol. 2001 Mar;49(3):377-83.
Elachouri et al., Genome Res. 2011 Jan;21(1):12-20.
Farkas et al., Biochemical Pharmacology, 2002,63, 921-932.
Fleischman RW et al., Thorax 1971; 26: 675-682.
Frick KD et al., (2012) Arch. Ophthalmol. 130, 629-634.
Giacomotto et al.,Hum Mol Genet. 2013 Nov 15;22(22):4562-78.
Halmosi et al. , Molecular Pharmacology, 2001,59(6),1497-1505.
Hata H et al., (2003) Jpn. J. Ophthalmol. 57, 259-262.
Hori et al., Genes Cells. 2011 May;16(5):527-44.
Kijas JW et al., (2002) Proc. Natl. Acad. Sci. U.S.A. 99, 6328-6333.
Kundu S et al., Int Med J 2013; 20: 34.
Lestienne et al., Biomed Pharmacother. 1994;48(5-6):199-214.
Lovy et al., J Vis Exp. 2012 Jul 20;(65):e3991.
Maarsingh H et al., Naunyn Schmiedebergs , Arch Pharmacol. 2008 Aug;378(2):171-84.
Marc RE et al., (2008) Mol. Vis. 14, 782-806.
Meersseman et al., Gene. 2014 Mar 1;537(1):154-63.
Mihaylova V et al., J Neurogenet. 2013 Jun;27(1-2):19-22.
Mishra et al.,Nat Rev Mol Cell Biol. 2014 Oct;15(10):634-46.
Naash ML et al., (1996) Invest. Ophthalmol. Vis. Sci. 37, 775-782.
Nagy et al., Toxicology and Applied Pharmacology 243. 96-103 (2010).
Nasrallah CM et al, Nat Rev Endocrinol. 2014 Nov;10(11):650-8.
Ozaki et al., Biochim Biophys Acta. 2012 Nov;1822(11):1783-95.
Papanicolaou et al., Am J Physiol Heart Circ Physiol. 2012 Aug 1;303(3):H243-55.
Paramasivam A et al., Mitochondrion. 2016 Jan;26:81-5.
Paskowitz et al. (2006) Br. J. Ophthalmol. 90, 1060-1066.
Plafker et al. Int Rev Cell Mol Biol. 2012;298:135-77.
Redmann et al., Int J Biochem Cell Biol. 2014 Aug;53:127-33.
Rossi L et al., Blood Cells Mol Dis 2004; 33: 57-63.
Rutar M et al., (2010) Curr. Eye Res. 35, 631-643.
Rutar M et al., (2011) Invest. Ophthalmol. Vis. Sci. 52, 5347-5358.
Schwarzer et al., J Physiol. 2014 Sep 1;592(17):3767-82.
Sharma et al., Circ Heart Fail. 2016 Apr;9(4):e002639.
Szabados et al., Biochemical Pharmacology. 2000, 59, 937-945.
Szczepanowska et al., Biochim Biophys Acta. 2012 Oct;1817(10):1740-6.
Wang et al., J Biol Chem. 2015 Jan 2;290(1):168-82.
Wenzel A et al., (2005) Prog. Retin. Eye Res. 24, 275-306.
Wert et al., Cell-Based Therapy for Retinal Degenerative Disease. Dev Ophthalmol.
Basel, Karger, 2014, vol 53, pp 33-43.
Wu et al., Development, 2015,142,681-691.
Young et al., Curr Opin Genet Dev. 2016 Apr 8;38:52-62.
Zanoni I et al., Nature. 2009 Jul 9;460(7252):264-8.
Zorzano et al., Front Aging Neurosci. 2015 Jun 10;7:101.

## Claims

1. The compound of formula I 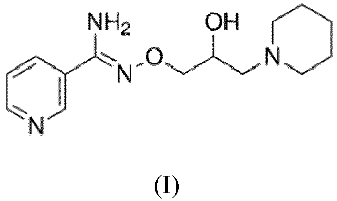 tautomers, enantiomers, and pharmaceutically acceptable salts thereof for use in the treatment of diseases characterised at least in part by an excessive mitochondrial fragmentation activity or by a reduced mitochondrial fusion activity.

2. The compound of claim 1, wherein the disease is pulmonary fibrosis.

3. The compound of claim 1, wherein the disease is age related macular degeneration.

4. The compound of claim 1, wherein the disease is retinitis pigmentosa.

5. The compound of claim 1, wherein the disease is heart failure with preserved ejection fraction.

6. The compound of claim 1, wherein the disease an inherited mtDNA depletion disorder.

7. The compound of claim 6, wherein the inherited mtDNA depletion disorder is Alper's Disease.

8. The compound of claim 1, wherein the disease is an inherited mtDNA deletion/mutation disorder.

9. The compound of claim 8, wherein the inherited mtDNA deletion/mutation disorder is progressive external ophthalmoplegia.

10. The compound of claim 8, wherein the inherited mtDNA deletion/mutation disorder is an ataxia-neuropathy spectrum disorder.

11. The compound of claim 8, wherein the inherited mtDNA deletion/mutation disorder is mitochondrial neurogastrointestinal encephalomyopathy.
